# EUROPEAN PATENT APPLICATION

(11) **EP 4 741 821 A1**
(43) Date of publication of application: **13.05.2026**
(21) Application number: 24306875.6
(22) Date of filing: 07.11.2024
(51) Int. Cl.: G01N 33/00, G01M 3/04

(54) **GAS DETECTION SYSTEM**

(71) Applicant: TotalEnergies OneTech, 92400 Courbevoie (FR)
(72) Inventor: BLANCO, Benoit, 92400 Courbevoie (FR); MAUNOURY, Abel, 92400 Courbevoie (FR)
(74) Representative: Bandpay & Greuter

(57) **Abstract**

The invention relates to a gas detection system comprising a plurality of cables (104, 105, 106) and an airborne body (100), wherein the airborne body (100) is attached to an anchored object (201) by the plurality of cables (104, 105, 106), the airborne body (100) carrying a sensor configured to take measurements for gas detection, wherein the system is configured so that the trajectory of the airborne body (100) is controllable by adjusting the length of one or more of the plurality of cables (104, 105, 106) using respective winches.

## Description

### Technical field

The present invention relates to a gas detection system that can provide monitoring of a gas such as methane (CH₄). The invention also relates to a process of gas detection using the gas detection system.

### Technical background

Anthropogenic methane emissions come from a large number of relatively small point sources. Methane emissions for example from oil and gas production units can be difficult to measure.

Typical existing solutions comprise atmospheric measurements from satellites, drones, cameras or ground sensors. All these technologies have different detection thresholds and revisit frequencies. Satellites can have a daily revisit and a detection threshold of 100 kg/h. Drones have a good detection threshold of 1 kg/h, but the revisit frequency depends on the availability of a drone pilot and is therefore generally low. In particular, currently, some legislations do not allow for autonomous drones. Ground sensors are poorly illuminated when there is a methane plume and generally not really efficient, and cameras have to focus on a specific portion.

Document CN103105468 discloses a kite and balloon air pollutant detection device. This kite is not controllable.

Within this context there is still a need for an improved system for *in situ* atmospheric gas detection having both frequent revisit and low gas detection threshold.

By way of background information, and without any relation to the context of atmospheric gas detection, document KR20190013376A relates to a helikite, which drifts with the wind and is not controllable. Furthermore, kite balloons such as the AirPup balloon are known.

### Summary of the invention

The invention relates to a gas detection system comprising a plurality of cables and an airborne body, wherein the airborne body is attached to an anchored object by the plurality of cables, the airborne body carrying a sensor configured to take measurements for gas detection, wherein the system is configured so that the trajectory of the airborne body is controllable by adjusting the length of one or more of the plurality of cables using respective winches.

In some variations, the airborne body comprises an envelope filled with a gas which is less dense than air, preferably helium.

In some variations, the envelope comprises a remotely operated security valve configured to release the gas from the envelope.

In some variations, the airborne body further comprises two wings on either side of the envelope.

In some variations, the plurality of cables comprise one or more main fixation cables and one or more steering cables, wherein the one or more steering cables are configured to control the orientation of the airborne body, and wherein, preferably, one steering cable is fixed to one wing of the airborne body and another steering cable is fixed to another wing of the airborne body.

In some variations, the gas detection system comprises a communication module, configured to send measurement data acquired by the sensor to a remote receiving system, wherein the communication module is preferably a wireless communication module, such as a Wi-Fi communication module.

In some variations, the gas detection system further comprises a positioning module, preferably including an altitude sensor and/or a GPS.

In some variations, the sensor is configured to detect methane.

In some variations, the anchored object is at sea.

In some variations, the gas detection system further comprises a motorized actuating device on the anchored object, configured for adjusting the length of one or more of the plurality of cables, and a control unit configured for controlling the motorized actuating device.

The invention also relates to a process of gas detection using the gas detection system as defined above, comprising driving the airborne body of the system along an airborne trajectory by adjusting the length of one or more of the plurality of cables and taking a plurality of gas measurements with the sensor.

In some variations, the trajectory comprises a plurality of portions of geodesics on a plurality of virtual spheres.

In some variations, the process further comprises sending measurement data from the sensor to a remote receiving system, preferably by wireless communication.

In some variations, the process is for monitoring and detecting a gas leak, preferably in the vicinity of an oil and/or gas production unit, more preferably in the vicinity of an offshore oil and/or gas production unit.

The invention relates to a gas detection system that provides an improved solution for detecting a gas such as methane in the atmosphere. This gas detection system can remain full-time on site and provide continuous atmospheric measurements. When compared with detection by drones, satellites, ground sensors and cameras, the present invention offers a better compromise in terms of detection threshold and revisit frequency.

The trajectory of the airborne body is controllable thanks to the plurality of cables which are connected to respective winches.

The invention has a low cost compared to traditional solutions. Indeed, the gas detection system is easy to deploy and to replace.

The invention can also be used on an industrial site and can be deployed for measuring a gas such as methane in the atmosphere even in the presence of wind at a speed of up to e.g., 25 knots. The airborne body's buoyancy prevents it from stalling and allows it to fly without wind.

### Brief description of the drawings

**FIGs. 1A****/****1B****/****1C****/****1D** are various views of the gas detection system according to a particular embodiment.
**FIG. 2** shows an artist view of the gas detection system according to a particular embodiment in the vicinity of a gas and/or oil production unit.
**FIG. 3** schematically shows a view of an example of trajectory (or portion of trajectory) for the airborne body of the gas detection system.
**FIG. 4** shows an example of winches as may be used in (or together with) the gas detection system.

### Detailed description

The invention will now be described in more detail without limitation in the following description.

An object of the present invention is a gas detection system comprising a plurality of cables and an airborne body, wherein the airborne body is attached to an anchored object by the plurality of cables, the airborne body carrying a sensor configured to take measurements for gas detection, wherein the system is configured so that a trajectory of the airborne body is controllable by adjusting the length of one or more of the plurality of cables.

By "anchored object" is meant an object which is either in a fixed position on or relative to the ground, or which is tethered to the ground (for example a buoy at sea, tethered to the bottom of the sea).

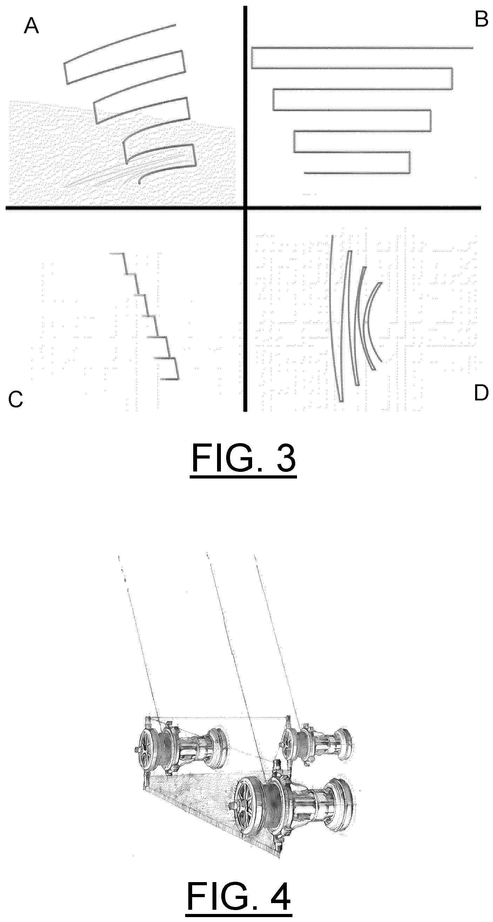

A plurality of winches may be fixed on the anchored object. Each cable may be connected to a respective winch. For example, each cable may be partly wound around a spool of the respective winch. By way of illustration, reference may be made to FIG. 4, which shows an example of three such winches.

The winches may be considered as part of the gas detection system. Alternatively, the gas detection system may be used with winches which are not part of the system itself.

The trajectory of the airborne body is said to be controllable if by adjusting the length of one or more of the plurality of cables, the airborne body can be displaced vertically, horizontally, or along any other atmospheric path, such as a line on a virtual sphere.

The sensor of the gas detection system (and any other electronic component on the airborne body, such as the modules which will be described in more detail below) may, in examples, be powered by one or more batteries mounted on the airborne body. The sensor of the gas detection system (and any other electronic component on the airborne body, such as the modules which will be described in more detail below) may in other examples be powered by solar panels fixed on the airborne body or may be powered by a power line connected to the anchored object.

The sensor of the gas detection system may provide an almost continuous revisit by taking measurements on a regular basis over a short period of time for example at least every second or at least every minute.

The sensor of the gas detection system may in examples be directly attached to the airborne body. It can be for example screwed, welded, stitched, glued or fixed by any other means. The sensor of the gas detection system may in examples be included in a dedicated compartment. This compartment needs to be open to air so that the sensor can take *in situ* measurements when the system is airborne.

The system may further comprise other modules configured to directly communicate with the sensor. For example, a control module may be present, configured to control the sensor, for example to instruct the sensor to take measurements or to cease taking measurements, to change a measurement rate, etc. The system may also comprise a storage module configured to store information, for example data from the sensor. The system may also comprise a processing module configured to process information, for example to process data from the sensor or from the storage module, and which may for example be configured to compute average values. The system may also comprise a communication module, configured to send measurement data from the sensor, from the storage module or from the processing module to a remote receiving system, which may be a computer or a server. The communication module may preferably be a wireless communication module and may be for example configured to operate according to the Wi-Fi protocol, the Bluetooth protocol, the WiMax protocol or any other type of wireless communication protocol. The communication module may also be configured to receive instructions from a remote emitting system (which may be the same as the remote receiving system or which may be different from the remote receiving system). The communication module may be configured to relay these instructions to the control module, the processing module, the storage module and/or the sensor so as to manage the operation of these components accordingly.

The measurement rate for the sensor may range for example from 0.01 Hz to 10 Hz, preferably from 0.1 Hz to 5 Hz, more preferably from 0.5 Hz to 2 Hz. The measurement rate may be fixed or variable. It may be set according to a program, as a function of user input (for example input acquired at a ground computer and sent to the communication module) and/or as a function of other operating parameters and/or sensed information; in particular, the measurement rate may depend on the real-time velocity of the airborne body and/or on sensed wind speed. Conversely, the velocity of the airborne body (which depends on wind speed and on the deployment/retraction of the cables) may be adjusted as a function of the measurement rate. As a result of either of these variants, the system may be configured to take measurements at predetermined distance intervals in the air, wherein these distance intervals may range for example from 50 cm to 20 m, preferably from 1 m to 15 m, more preferably from 2 m to 10 m, even more preferably from 3 m to 7 m.

The communication module may send the measurements acquired from the sensor at a fixed rate, for example at least every second, at least every minute, at least every hour or at least every day.

In some embodiments, the system comprises a positioning module mounted on the airborne body. The positioning module may comprise an altitude sensor, such as an accelerometer. The positioning module may preferably comprise or be a GPS. Positioning information may also be stored in the storage module and/or processed in the processing module and/or sent *via* the communication module to the remote receiving system.

In some embodiments, the gas detection system comprises wind sensing equipment. The wind sensing equipment may be onboard, *i.e*., mounted on the airborne body and/or may be included in a ground station, for example on the anchored object. The wind sensing equipment may be configured to measure wind speed and/or wind direction. Information from the wind sensing equipment may also be stored in the storage module and/or processed in the processing module and/or sent *via* the communication module to the remote receiving system and/or to the control unit which will be described in more detail below.

The control module, storage module, processing module, communication module, positioning module (if present), onboard wind sensing equipment (if present) may be fully distinct or may be partly or fully integrated together. For example, part or all of these modules may be integrated as an on-board computer. Besides, each of these modules may be integrated together with the sensor to form a sensing device capable of carrying out the actions described above. Conversely, one or more, or all of these modules may be devices which are external to the sensor and which may communicate with the sensor and/or which each other. In this case, these modules are preferably contained in the same compartment (described above) as the sensor.

The airborne body may have a maximum dimension of for example 1 to 5 m, preferably 2 to 4 m, such as approximately 3 m.

The sensor may be elongated. For example, the sensor may have a length (maximal dimension) from 50 cm to 1.5 m, preferably from 70 cm to 1 m. The sensor is typically located on an inferior surface of the airborne body (configured to face the ground when airborne), along a longitudinal axis of the airborne body. Such a long sensor could not be readily carried by a drone having a conventional size.

The sensor is preferably lightweight, in examples, it may weigh from 20 g to 1 kg, preferably from 50 to 200 g, such as approximately 100 g. Such a sensor is readily available at low cost.

The sensor of the gas detection system may be configured to detect any gas (including volatile substances) in the atmosphere, such as methane or carbon dioxide. In some cases, the sensor may be able to detect more than one gas. Preferably, the sensor is a methane sensor.

The sensor of the gas detection system may perform atmospheric measurement thanks to the airborne body's trajectory having a gas detection threshold, for example, below 10 kg/h. More preferably, the gas detection threshold may be below 5 kg/h. The gas detection threshold may be for example from 0.1 to 10 kg/h, or from 0.5 to 5 kg/h.

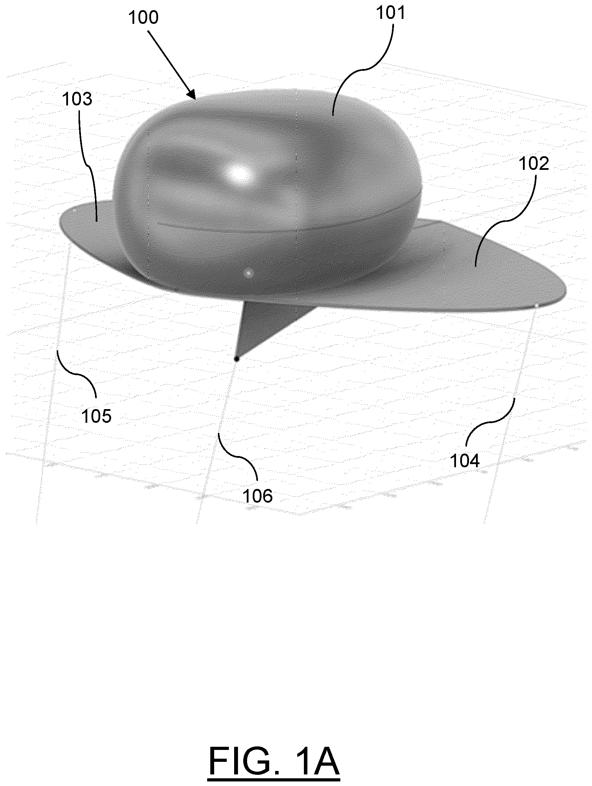

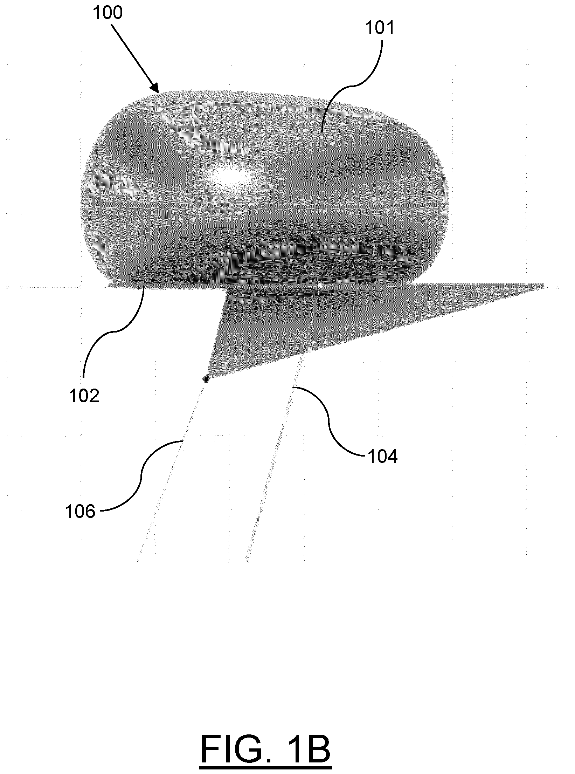

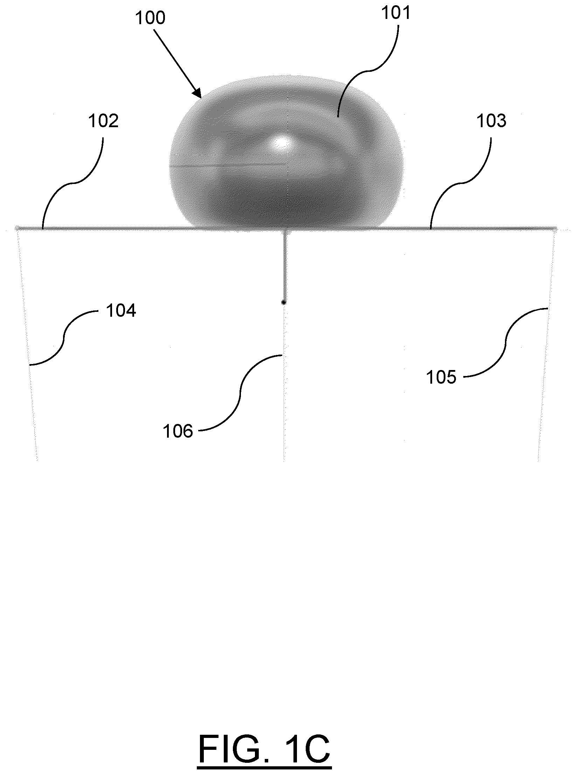

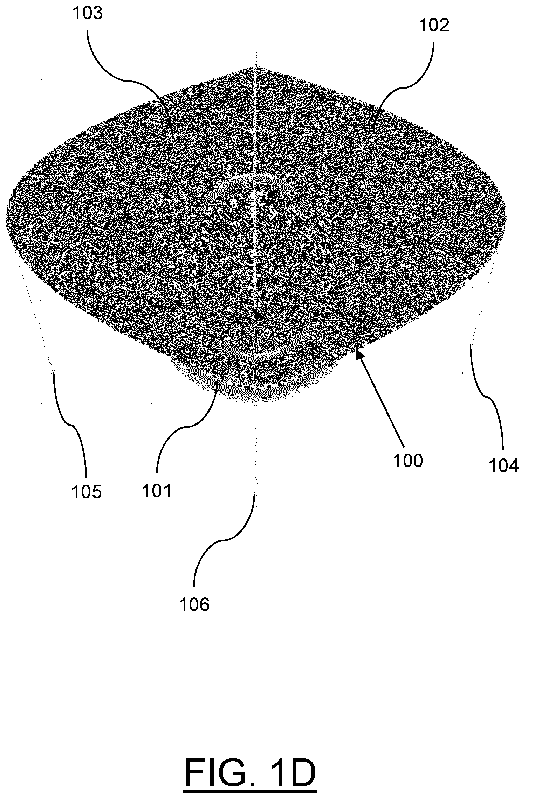

One example of the gas detection system according to a particular embodiment of the invention is shown in FIGs. 1A, 1B, 1Cand 1D. In this example, the airborne body 100 is attached to an anchored object (not shown here) by a plurality of cables 104, 105, and 106. The airborne body comprises an envelope 101 filled with a gas which is less dense than air. The volume of the envelope and the gas in the envelope may be selected to ensure that, even when there is little or no wind, the airborne body 100 may remain airborne. For example, the envelope may be filled with helium. In examples, the envelope 101 may further comprise a remotely operated security valve (not shown here) configured to release the gas from the envelope 101. The remotely operated security valve can release the gas during emergency cases caused by climate hazards such as floods, storms, hurricanes, typhoons, cyclones, or simply wind speeds over 40 knots, in order to facilitate retrieval of the gas detection system in such an emergency case. The remotely operated security valve may be controlled by the control module and/or by the communication module as described above, or may have a dedicated control and/or communication capability.

The airborne body 100 may preferably be made of fabric or carbon fiber. In examples, the envelope 101 of the airborne body 100 may comprise a double layer (or a multiple layer) of material to be more resistant to high wind speeds and climate events (violent gusts, squalls, heavy rains, ...).

The airborne body 100 may also comprise a first wing 102 on one side of the envelope 101 and a second wing 103 on another side of the envelope 101, for example relative to the longitudinal axis defined above. The airborne body 100 may alternatively comprise no wing, one wing, two wings or a plurality of wings on the sides of the envelope 101. In case there are a plurality of wings, the wings may be the same in size, shape and materials or the wings may be different.

By wing is meant a portion which does not contain a gas (contrary to the envelope) and which extends from the envelope. In some cases, the wing may be substantially planar or otherwise substantially two-dimensional (*i.e*. having a negligible thickness in comparison with the overall dimensions of said wing).

The airborne body 100 may have a symmetrical structure relative to a symmetry plane comprising the longitudinal axis. In particular the first wing 102 may be symmetrical with the second wing 103. More generally, when two or more wings are present they may be symmetrically arranged. The wings may be substantially planar.

The airborne body 100 may be attached on to the anchored object by a main fixation cable 106. The main fixation cable 106 may be fixed on the inferior surface (as defined above) of the envelope 101. As illustrated, the main fixation wing 106 may be fixed to the envelope 101 *via* an intermediate flap which may for example extend perpendicularly to the inferior surface of the envelope 101 (and/or may be oriented perpendicularly to the wings).

The first wing 102 may be attached to the anchored object by a first steering cable 104. The second wing 103 may be attached to the anchored object by a second steering cable 105. The airborne body 100 may be attached to the anchored object by more than a main fixation cable and two steering cables. In examples, the airborne body 100 may be attached to the anchored object by a plurality of main fixation cables and/or a plurality of steering cables. In this example, there is preferably at least one steering cable fixed to each wing of the airborne body 100. There may also be a plurality of main fixation cables fixed to any part of the airborne body 100. Each one of the cables may be fixed by one or more anchoring points onto the airborne body 100.

The main fixation cable may be for example fixed closer to the front of the envelope, while the steering cables may be for example fixed closer to the rear of the envelope. "Front" and "rear" may refer to opposite ends along the longitudinal axis.

The main function of the main fixation cable(s) is to assume most of the tension between the airborne body 100 and the anchored object. The main function of the steering cable(s) is to modify the orientation and/or trajectory of the airborne body 100.

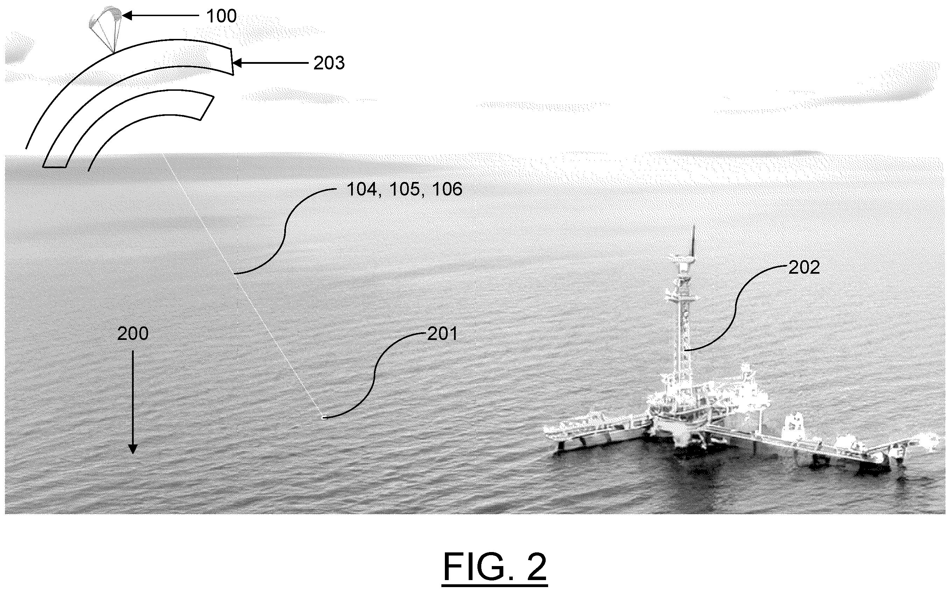

One example of the gas detection system according to a particular embodiment of the invention is shown in FIG. 2. The gas detection system is attached to the anchored object 201 by a plurality of cables 104, 105 and 106 as described above. The anchored object 201 may be a buoy at sea 200 but in other embodiments, the gas detection system may also be used above land and in this case, the anchored object may be directly fixed on the ground. The gas detection system may further comprise a motorized actuating device on the anchored object 201, configured for adjusting the length of one or more of the plurality of cables 104, 105 and 106, and a control unit configured for controlling the motorized actuating device. The control unit may receive input instructions from a user. Alternatively, the control unit may be configured to autonomously control the motorized actuating device according to a predefined program. The control unit may receive and process information from the wind sensing equipment if present and/or may receive and process information indicative of the airborne body velocity.

The motorized actuating device may comprise a motor for each winch of the plurality of winches such that each motor actuates a winch of the plurality of winches. For example, activating the motor may deploy or retract (adjust the length of) one or more cables of the plurality of cables 104, 105 and 106.

When the airborne body 100 needs to be recovered for maintenance or in emergency cases, such as in case of high wind speeds (e.g. over 40 knots), all motors of the motorized actuating device may be activated. The airborne body 100 should preferably be brought back to the anchored object in less than a minute. This recovery of the airborne body may be carried out automatically based on a measurement of wind speed by the wind sensing equipment described above.

Another object of the invention is a process of gas detection using the gas detection system comprising driving the airborne body 100 of the system along an airborne trajectory 203 by adjusting the length of one or more of the plurality of cables 104, 105 and 106, and taking a plurality of gas measurements with the sensor. The airborne trajectory may be similar to the trajectory of a drone. The flight window of the airborne body 100 may typically be, in examples, a quarter-sphere.

The process may comprise the use of the motorized actuating device to control the airborne body 100. In this example, to move horizontally, vertically or along any other atmospheric path, such as a line on a virtual sphere, one or more motors of the motorized actuating device is/are activated to operate one or more winches, and therefore to retract or deploy one or more respective cable(s) fixed to the anchored object. In some cases, the retraction or deployment may be minimal, so as to simply adjust the tension on the cable without significantly modifying the distance between the anchoring point(s) of the cable on the airborne body and the anchored object. In some examples, when there are two steering cables, one for each of two wings on either side of the envelope of the airborne body, by retracting one of the steering cable, it may be possible to rotate the airborne body in one direction, and by retracting the other steering cable, it may be possible to rotate the airborne body in the opposite direction. It may also be possible to simultaneously act on both steering cables, for example by simultaneously retracting one steering cable and deploying the other steering cable so as to effect rotation of the airborne body in one direction.

Referring back to FIG. 2, the airborne trajectory 203 may comprise portions having different altitudes. To reduce or increase the altitude, or to reduce or increase the distance between the airborne body and the anchored object, a motor of the motorized actuating device may be activated to actuate the winch connected to the at least one main fixation cable. This airborne trajectory 203 may comprise portions circumscribed in different virtual spheres having different radii. For example, it may comprise a portion circumscribed in a virtual (quarter) sphere of a first radius *r*₁, another portion circumscribed in a virtual (quarter) sphere of radius *r*₂, with *r*₂ < *r*₁, another portion circumscribed in a virtual (quarter) sphere of radius *r*₃, with *r*₃ < *r*₂, etc. The airborne body may transition from one portion to the next portion by modifying the length of the at least one main fixation cable, or by simultaneously modifying the length of all cables, so as to modify the altitude or the distance between the anchored object and the airborne body (again by activating one or more motors of the motorized actuating device to actuate one or more winches). For example, the airborne trajectory may further comprise a plurality of portions of geodesics on a plurality of virtual spheres or may comprise a plurality of straight lines.

FIG. 3 shows an illustration of (part of) the airborne trajectory on a virtual sphere. View A is a three-dimensional illustration, view D is a top view (vertical direction of viewing), views B and C are respectively a front view and a left view (two perpendicular horizontal directions of viewing).

In other examples, the airborne trajectory may be comprised in a plane. This may be achieved by continuously modifying the length of the fixation cable, and possibly of all cables.

The airborne body 100 may be repeatedly driven along the same trajectory, optionally in both directions, for example by repeated sequences of increasing the altitude and decreasing the altitude; or by repeated sequences of increasing the distance between the anchored object and the airborne body and decreasing the distance between the anchored object and the airborne body. Measurements for gas detection may be made on part or all of the trajectory, for example as the airborne body progressively ascends, or as the airborne body progressively descends, or both as the airborne body progressively ascends and progressively descends.

The airborne body 100 may also be successively driven along different trajectories, optionally depending on meteorological conditions.

The trajectory of the airborne body may extend to a maximum distance of from 50 m to 500 m, preferably from 100 m to 400 m, from the anchored object. This makes it possible to detect a potential gas leak plume.

If the wind speed becomes undesirably high, the process of gas detection may be stopped. The motorized actuating device may further comprise a security implementation wherein the winches are configured to withdraw the airborne body if a condition is met. The condition may be related to the meteorological conditions, or related to a criterion based on the measurements acquired by the sensor.

The gas detection system may comprise a docking station, which may be placed on the anchored object. The docking station may be configured to store the airborne body. The docking station may comprise a charging unit for charging one or more batteries mounted on the airborne body. The docking station may be configured for automated launch or takeoff and automated recovery or landing of the airborne body.

The process of gas detection may be used for monitoring and detecting a possible gas leak, preferably in the vicinity of an oil and/or gas production unit, more preferably in the vicinity of an offshore oil and/or gas production unit 202. When the anchored object 201 is at sea 200, the anchored object 201 is preferably a dedicated buoy. Alternatively, the anchored object could be part of the oil and/or gas production unit 202, if allowed by regulations. The airborne body 100 is preferably configured to fly downwind from the oil and/or gas production unit 202 based on the direction of prevailing winds. For example the anchored object may be located downwind from the oil and/or gas production unit 202 based on the direction of prevailing winds.

## Claims

1. A gas detection system comprising a plurality of cables (104, 105, 106) and an airborne body (100), wherein the airborne body (100) is attached to an anchored object (201) by the plurality of cables (104, 105, 106), the airborne body (100) carrying a sensor configured to take measurements for gas detection, wherein the system is configured so that the trajectory of the airborne body (100) is controllable by adjusting the length of one or more of the plurality of cables (104, 105, 106) using respective winches.

2. The gas detection system of claim 1, wherein the airborne body (100) comprises an envelope (101) filled with a gas which is less dense than air, preferably helium.

3. The gas detection system of claim 2, wherein the envelope (101) comprises a remotely operated security valve configured to release the gas from the envelope (101).

4. The gas detection system of claim 2 or 3, wherein the airborne body (100) further comprises two wings (102, 103) on either side of the envelope (101).

5. The gas detection system of any of claims 1-4, wherein the plurality of cables (104, 105, 106) comprise one or more main fixation cables (106) and one or more steering cables (104, 105), wherein the one or more steering cables (104, 105) are configured to control the orientation of the airborne body (100), and wherein, preferably, one steering cable (104) is fixed to one wing (102) of the airborne body (100) and another steering cable (105) is fixed to another wing (103) of the airborne body (100).

6. The gas detection system of any of claims 1-5, which comprises a communication module, configured to send measurement data acquired by the sensor to a remote receiving system, wherein the communication module is preferably a wireless communication module, such as a Wi-Fi communication module.

7. The gas detection system of any of claims 1-6, which further comprises a positioning module, preferably including an altitude sensor and/or a GPS.

8. The gas detection system of any of claims 1-7, wherein the sensor is configured to detect methane.

9. The gas detection system of any of claims 1-8, wherein the anchored object (201) is at sea (200).

10. The gas detection system of any of claims 1-9, further comprising a motorized actuating device on the anchored object (201), configured for adjusting the length of one or more of the plurality of cables (104, 105, 106), and a control unit configured for controlling the motorized actuating device.

11. A process of gas detection using the gas detection system of any of claims 1-10, comprising driving the airborne body (100) of the system along an airborne trajectory (203) by adjusting the length of one or more of the plurality of cables (104, 105, 106) and taking a plurality of gas measurements with the sensor.

12. The process of claim 11, wherein the trajectory comprises a plurality of portions of geodesics on a plurality of virtual spheres.

13. The process of claim 11 or 12, further comprises sending measurement data from the sensor to a remote receiving system, preferably by wireless communication.

14. The process of any of claims 11-13, for monitoring and detecting a gas leak, preferably in the vicinity of an oil and/or gas production unit, more preferably in the vicinity of an offshore oil and/or gas production unit (202).
